(19) Europäisches Patentamt
European Patent Office
Office européen des brevets

(11) Veröffentlichungsnummer: **0 010 717**
**B1**

(12) **EUROPÄISCHE PATENTSCHRIFT**

(45) Veröffentlichungstag der Patentschrift: **21.09.83**

(21) Anmeldenummer: **79104083.5**

(22) Anmeldetag: **22.10.79**

(51) Int. Cl.³: **C 07 D 267/12,** A 61 K 31/55 // C07C97/07, C07C91/23, C07C103/38

# (54) Neue 6,7-Cycloalkano-1,4-oxazepine, Verfahren zu deren Herstellung und diese enthaltende Arzneimittel.

(30) Priorität: **26.10.78 DE 2846567**

(43) Veröffentlichungstag der Anmeldung: **14.05.80 Patentblatt 80/10**

(45) Bekanntmachung des Hinweises auf die Patenterteilung: **21.09.83 Patentblatt 83/38**

(84) Benannte Vertragsstaaten: **AT BE CH DE FR GB IT LU NL SE**

(56) Entgegenhaltungen: **DE-A-2 609 601**

(73) Patentinhaber: **BASF Aktiengesellschaft, Carl-Bosch-Strasse 38, D-6700 Ludwigshafen (DE)**

(72) Erfinder: **Treiber, Hans Joerg, Dr., Erzbergerstrasse 40, D-6831 Bruehl (DE)**
Erfinder: **Lenke, Dieter, Dr., Kekuléplatz 1, D-6700 Ludwigshafen (DE)**
Erfinder: **Worstmann, Wolfgang, Dr., Am Bildstock 11, D-6717 Gruenstadt (DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Neue 6,7-Cycloalkano-1,4-oxazepine, Verfahren zu deren Herstellung und diese enthaltende Arzneimittel

Es ist bereits aus der DE-C-1620198 bekannt, dass bestimmte Benzoxazocine eine analgetische Wirkung besitzen. Die bekannteste dieser Verbindungen ist das Nefopam (5-Methyl-1-phenyl-3,4,5,6-tetrahydro-1H-benz[f]-2,5-oxazocin).

Es wurden nun Verbindungen gefunden, die das Nefopam in ihrer Wirksamkeit deutlich übertreffen.

Gegenstand der Erfindung sind 6,7-Cycloalkano-1,4-oxazepine der allgemeinen Formel I

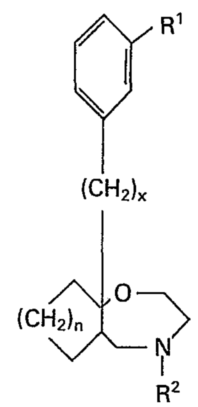

I,

worin $R^1$ ein Wasserstoffatom, eine Hydroxygruppe oder eine Alkoxy- oder Acyloxygruppe mit 1–4 Kohlenstoffatomen, $R^2$ einen Methyl-, Ethyl- oder Allylrest, n die Zahl 1, 2 oder 3 und x die Zahl 0 oder 1 bedeutet, sowie deren Salze mit physiologisch verträglichen Säuren.

Gegenstand der Erfindung ist weiter ein Verfahren zur Herstellung der 6,7-Cycloalkano-1,4-oxazepine der allgemeinen Formel I, welches darin besteht, dass man eine Verbindung der allgemeinen Formel II

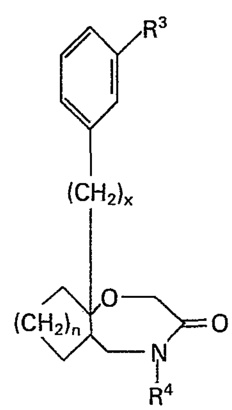

II,

worin $R^3$ ein Wasserstoffatom oder eine Alkoxygruppe mit 1–4 Kohlenstoffatomen, $R^4$ einen Methyl-, Ethyl- oder Allylrest oder eine Benzylgruppe darstellen und x und n die oben angegebene Bedeutung besitzen, reduziert und anschliessend gegebenenfalls eine Alkoxygruppe gegen eine Hydroxy- oder Acyloxygruppe und/oder eine Benzylgruppe gegen einen Kohlenwasserstoffrest austauscht und die so erhaltenen Verbindungen, falls gewünscht, in ihre Salze mit physiologisch verträglichen Säuren überführt.

Schliesslich betrifft die Erfindung auch Arzneimittel, welche 6,7-Cycloalkano-1,4-oxazepine der allgemeinen Formel I enthalten, sowie die neuen Substanzen zur Verwendung bei der Bekämpfung von Schmerzzuständen.

Die Verbindungen I enthalten zwei asymmetrische Kohlenstoffatome, so dass sie grundsätzlich zwei diasteromere Reihen bilden können. Die vorliegende Erfindung betrifft lediglich solche Verbindungen, in denen beide Ringe miteinander cisverknüpft sind. Die neuen Verbindungen lassen sich sowohl in Form ihrer Racemate als auch ihrer Antipoden darstellen.

Zur Reduktion der Verbindungen II benötigt man starke Reduktionsmittel wie Diboran oder vorzugsweise Lithiumaluminiumhydrid. Als Lösungsmittel eignen sich besonders Tetrahydrofuran sowie Dimethoxyethan, Dioxan und Ether.

Die Reduktion erfolgt bei erhöhter Temperatur, vorzugsweise der Siedetemperatur des Lösungsmittels.

Ein Benzylrest in der 4-Stellung lässt sich leicht durch katalytische Hydrierung entfernen. Die Hydrierung kann in Alkoholen, wie Methanol oder Ethanol, oder Essigsäure als Lösungsmittel vorzugsweise bei Raumtemperatur mit Edelmetallen der 8. Hauptgruppe des Periodensystems, vorzugsweise Pd/Kohle, durchgeführt werden.

Das so erhaltene sekundäre Amin kann mit einem Alkylhalogenid $R^4$-Hal in Gegenwart eines Säurefängers wie Alkalicarbonat, Alkalihydroxid oder einem tertiären Amin in einem Lösungsmittel wie Methylisobutylketon bei einer Temperatur von etwa 0–150°C, vorzugsweise etwa 80°C alkyliert werden. Die Alkylierung kann auch durch Umsetzen mit einer Carbonsäure und anschliessende Reduktion der C=O-Gruppe mit Lithiumaluminiumhydrid erfolgen.

Der Austausch der Methoxygruppe gegen eine Hydroxygruppe kann z.B. mit Natriummethylmercaptid in einem dipolar aprotischen Lösungsmittel wie Hexamethylphosphorsäuretriamid, Dimethylsulfoxid oder Dimethylformamid bei 50–200, vorzugsweise 80–150°C erfolgen.

Zur Acylierung der freien Hydroxygruppen können praktisch alle bekannten Verfahren herangezogen werden. Am einfachsten ist die Umsetzung mit den Säureanhydriden der -halogeniden bei erhöhter Temperatur.

Die zur Herstellung der neuen Verbindungen benötigten Ausgangsstoffe der allgemeinen Formel II sind noch nicht beschrieben. Sie lassen sich wie folgt herstellen:

Aus Cycloalkanonen, Formaldehyd und sekundären Benzylaminderivaten werden sogenannte Mannich-Verbindungen der Formel III hergestellt (vgl. Org. Reactions I (1942), GB-PS 615136, J. Chem. Soc. 1950, 1512, J. Org. Chem. 24, 1069 (1959)):

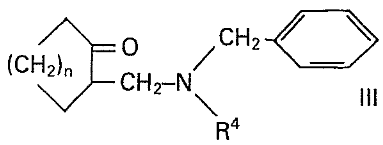

III

Durch Umsetzen von III mit entsprechenden Grignard-Verbindungen erhält man die Verbindungen IV (vgl. J. Am. Chem. Soc. 71, 2050 (1949), GB-PS 997399, DE-PS 1199764, Arzneim. Forsch. 28, 107 (1978)):

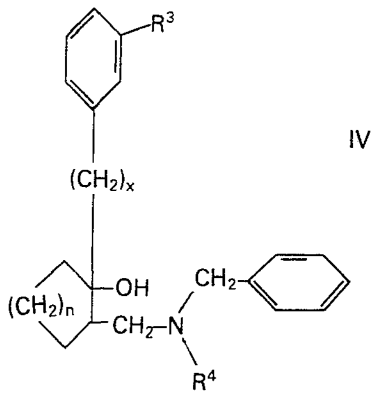

IV

aus denen der Benzylrest abhydriert wird. Aus der so erhaltenen Verbindung erhält man durch Umsetzen mit Chloracetylchlorid in Gegenwart von verdünnter Natronlauge oder Triethylamin die Verbindung V

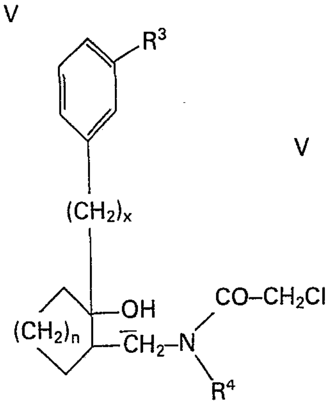

V

Aus V lassen sich durch Erwärmen mit einer Base wie Kalium-tert.-butanolat in Dimethylsulfoxid die Verbindungen II herstellen.

In der Verbindung V stehen die freie OH-Gruppe und die $CH_2$–$NR^4$–CO–$CH_2Cl$-Gruppe zueinander in der cis-Stellung, wenn diese in der angegebenen Weise hergestellt werden.

Will man die Verbindung I in Form ihrer optischen Antipoden herstellen, so ist es zweckmässig, die Racemattrennung bereits an den Verbindungen IV vorzunehmen.

Die erfindungsgemässen Substanzen zeichnen sich durch eine ausgeprägte analgetische Wirkung aus. Sie eignen sich daher zur Pharmakotherapie von Schmerzzuständen verschiedener Ursache.

Als Modell zur Prüfung der analgetischen Wirkung diente der sogenannte Brennstrahl-Test nach D'AMOUR und SMITH (J. Pharmacol. 72, 74–79, 1941). In diesem Experiment werden die zu prüfenden Substanzen (wässrige Lösungen; Injektionsvolumen 10 ml/kg) Gruppen von 10 weiblichen Mäusen (Stamm: NMRI) im Gewicht von 20–22 g intraperitoneal oder oral appliziert.

Schmerzreaktionen werden durch thermische Reizung (fokussierte Wärmebestrahlung des Schwanzes mit einer Halogenlampe für maximal 30 sec) vor und 30 min nach der Substanz-Applikation ausgelöst.

Die Zeit bis zum reflektorischen Wegziehen des Schwanzes aus der Bestrahlungszone wird als Reaktionszeit gemessen. Sie beträgt bei 670 unbehandelten Tieren 6,5 ± 0,29 sec.

Analgetisch wirkende Substanzen verlängern die Reaktionszeit dosisabhängig. Zwischen der Logarithem der Dosen (mg/kg) und der relativen Verlängerung der Reaktionszeit (△ %) besteht eine lineare Beziehung, aus der mit Hilfe der Regressionsanalyse als ED 100% die Dosis berechnet wird, welche die Reaktionszeit verdoppelt. Bei einer Bestrahlungsdauer von maximal 30 sec beträgt die grösstmögliche Verlängerung der Reaktionszeit rund 360%.

Sowohl nach intraperitonealer als auch nach oraler Applikation kann eine dosisabhängige analgetische Wirkung nachgewiesen werden (Tabelle 1). Die neuen Substanzen sind hier dem bekannten Analgeticum Nefopam (5-Methyl-1-phenyl-3,4,5,6-tetrahydro-1H-2,5-benzoxazocin) deutlich überlegen, besonders bei der pharmakotherapeutisch wichtigen oralen Applikation. Die erfindungsgemässen Substanzen zeichnen sich hier durch eine etwa 2- bis 5-fach stärkere Wirksamkeit aus. Die höhere orale Wirksamkeit geht mit einer Zunahme der für die Sicherheit der Anwendung ausserordentlich wichtigen relativen enteralen Wirksamkeit einher. Die enterale Wirksamkeit wird durch den Quotienten aus den wirksamen Dosen (ED 100%) bei intraperitonealer und bei oraler Applikation ausgedrückt. Sie liegt bei den neuen Substanzen zwischen 0,35 und 0,96 und ist damit 2,1–5,6mal grösser als die des Nefopam.

Unter den Bedingungen des Brennstrahl-Tests kann die Reaktionszeit durch die erfindungsgemässen Verbindungen bei beiden Applikationsarten um maximal 275–416% (i.p.) bzw. 280–297% (per os) verlängert werden, ohne dass Todesfälle infolge toxischer Substanzwirkungen auftreten. Dagegen sind beim Nefopam nur Verlängerungen von maximal 192% (21,5 mg/kg i.p.) bzw. 93% (46,4 mg/kg per os) möglich. Höhere Dosen (46,4 mg/kg i.p. bzw. 100 mg/kg per os) wirken bereits letal und töten 60% der behandelten Tiere (Tabelle 1).

Die vergleichsweise geringe Toxizität der neuen Substanz lässt sich auch aus den akuten letalen Dosen (LD 50) bei i.p. Applikation ableiten (Tabelle 2). Die LD 50-Werte liegen um etwa 90% bis 240% höher als die des Nefopam.

Tabelle 1

| Substanz des Beispiels Nr. | Applikation i.p. ED 100% 1) | relative Wirksamkeit | Max. Wirkung 2) mg/kg | % | Applikation per os ED 100% | relative Wirksamkeit | Max. Wirkung 3) mg/kg | △ % | Q 3) |
|---|---|---|---|---|---|---|---|---|---|
| 3 | 11,4 | 0,71 | 46,4 | 416 | 17,0 | 2,73 | 100 | 397 | 0,67 |
| 6 | 4,66 | 1,73 | 46,4 | 380 | 9,49 | 4,89 | 46,4 | 364 | 0,49 |
| 7 | 6,55 | 1,23 | 46,4 | 364 | 18,6 | 2,50 | 46,4 | 332 | 0,35 |
| 9 (−) | 11,0 | 0,74 | 46,4 | 348 | 17,6 | 2,64 | 100 | 397 | 0,63 |
| 9 (+) | 16,7 | 0,48 | 46,4 | 249 | 23,5 | 1,97 | 100 | 370 | 0,71 |
| 7 β | 12,6 | 0,64 | 46,4 | 275 | 13,1 | 3,54 | 100 | 280 | 0,96 |
| Nefopam | 8,08 | ≡ 1,00 | 46,4 4) | 192 | 46,4 | 1,00 | 46,4 5) | 93 | 0,17 |

1) Dosis (mg/kg), welche die Reaktionszeit um 100% verlängert
2) Grösstmögliche Verlängerung der Reaktionszeit bei einem Dosenabstand von $\sqrt[3]{10}$
3) $Q = \text{Enterale Wirksamkeit} = \frac{\text{ED 100\% i.p.}}{\text{ED 100\% per os}}$
4) Bei 46,4 mg/kg 6 von 10 Tieren gestorben
5) Bei 100 mg/kg 6 von 10 Tieren gestorben

Tabelle 2

| Substanz des Beispiels Nr. | LD 50 mg/kg | relative Toxizität |
|---|---|---|
| 3 | 110 | 0,52 |
| 6 | 127 | 0,45 |
| 7 | 165 | 0,35 |
| 9 (−) | 145 | 0,40 |
| 9 (+) | 110 | 0,52 |
| 7 β | 194 | 0,30 |
| Nefopam | 57,3 | ≡ 1,00 |

Die erfindungsgemässen Verbindungen können in üblicher Weise oral oder parenteral (intravenös, intramuskulär) verabfolgt werden.

Die Dosierung hängt vom Alter, Zustand und Gewicht des Patienten sowie von der Applikationsart ab. In der Regel beträgt die tägliche Wirkstoffdosis zwischen etwa 0,1 und 2,0 mg/kg Körpergewicht bei oraler Gabe und zwischen etwa 0,05 und 1,0 mg/kg Körpergewicht bei parenteraler Gabe. Im Normalfall werden mit täglichen Dosen von 0,3–1,5 mg/kg oral und 0,1–0,5 mg/kg parenteral zufriedenstellende Ergebnisse erzielt.

Die neuen Verbindungen können in den gebräuchlichen galenischen Applikationsformen fest oder flüssig angewendet werden, z.B. als Tabletten, Kapseln, Pulver, Granulate, Dragees, Lösungen, oder Suppositorien. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fliessreguliermitteln, Weichmachern, Netzmitteln, Dispergiermitteln, Emulgatoren, Lösungsmitteln, Retardierungsmitteln und/oder Antioxidantien verarbeitet werden (vgl. L.G. Goodman, A. Gilman: The Pharmacological Basis of Therapeutics).

Die neuen Substanzen können auch in Form ihrer Salze mit physiologisch verträglichen Säuren verabfolgt werden. Solche Säuren sind beispielsweise Salzsäure, Schwefelsäure, Phosphorsäure, Weinsäure, Zitronensäure, Fumarsäure, Essigsäure, Ameisensäure, Bernsteinsäure, Maleinsäure, Milchsäure, Amidosulfonsäure.

Herstellung der Ausgangsstoffe

a) 2-(N-Benzylmethylamino)-methyl-cyclopentanon-hydrochlorid

87,5 g (0,55 Mol) N-Benzylmethylamin-hydrochlorid werden mit 84 g (1,0 Mol) Cyclopentanon, 45 g Paraformaldehyd (≙ 1,5 Mol Formaldehyd) und 500 ml Ethanol eine Stunde unter Rühren und Rückfluss zum Sieden erhitzt, wobei Lösung eintritt. Nach Abkühlen wird das Lösungsmittel im Vakuum abdestilliert, der ölige Rückstand in 100 ml Isopropanol gelöst und danach mit 500 ml Essigsäureethylester versetzt, wobei nach einiger Zeit das Produkt auskristallisiert.
Ausbeute 75 g (54% der Theorie)
Fp. 129–130°C

In ähnlicher Weise wurden erhalten:

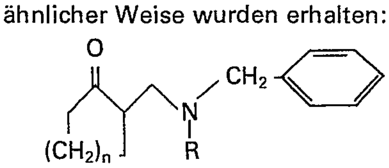

| n | R | Hydrochlorid Fp. (°C) | Ausbeute |
|---|---|---|---|
| 2 | $CH_3$ | 140 | 83% |
| 2 | $CH_2$- 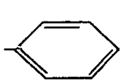 | 250–251 | 48% |
| 3 | $CH_3$ | 120–123 | 43% |

b) 2-(N-Benzylmethylamino)methyl-1-(3-methoxybenzyl)-cyclohexanol

In eine Grignardlösung, die man aus 110 g (0,7 Mol) 3-Methoxybenzylchlorid 16,8 g Magnesium und 700 ml trockenem Ether bereitet hat, trägt man unter Rühren und unter Kühlung innerhalb 30 min 67 g (0,25 Mol) 2-(N-Benzylmethylamino)-methylcyclohexanon-hydrochlorid (erhalten gemäss a) ein, lässt 16 Stunden bei Raumtemperatur nachrühren, zersetzt den Reaktionsansatz mit einem Überschuss an konzentrierter Ammoniumchloridlösung, trennt die Etherschicht ab, trocknet mit Natriumsulfat, verjagt den Ether und destilliert den Rückstand im Vakuum.
Ausbeute: 60g (68% der Theorie)
Kp. 200–205°C/0,007 mbar

In ähnlicher Weise wurden hergestellt:

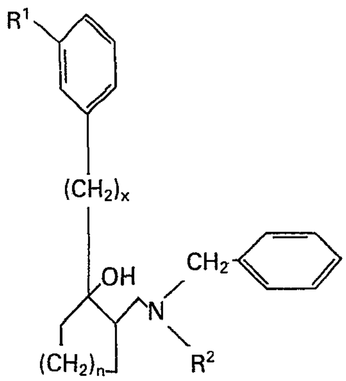

| $R^1$ | $R^2$ | x | n | Ausbeute % | Kp (°C)/mbar |
|---|---|---|---|---|---|
| $CH_3O$ | $CH_3$ | 0 | 1 | 54 | 220/0,026 |
| $CH_3O$ | $CH_3$ | 0 | 2 | 81 | 220–230/0,13 |
| $CH_3O$ | $CH_3$ | 1 | 2 | 68 | 180–190/0,13 |
| H | $CH_3$ | 0 | 2 | 87 | 180–185/0,7 |
| $CH_3O$ | $CH_3$ | 0 | 2 | 52 | 240–260/0,07 |

Racematspaltung von 2-(N-Benzylmethylamino)methyl-1-(3-methoxy-phenyl)-cyclohexanol

Aus einer Lösung von 150g (0,44 Mol) 2-(N-Benzylmethylamino)-methyl-1-(3-methoxy-phenyl)-cyclohexanols und 168g (0,44 Mol) L(-)-0,0-Dibenzoylweinsäure-Monohydrat in 1000 ml Isopropanol kristallisiert das Dibenzoyltartrat der linksdrehenden Base aus. Nach weiterem zweimaligem Umkristallisieren aus der jeweils 3fachen Menge Isopropanol erhält man 130g (86% der Theorie) drehwertkonstantes Produkt.
Spezifischer Drehwert: $[\alpha]_D^{20} = -77°$ (Methanol, C = 27 mg/ml)

Aus dem Salz erhält man in üblicher Weise die Base mit dem spezifischen Drehwert:
$[\alpha]_D^{20} = -89°$ (Methanol, C = 22 mg/ml).

Verwendet man für die Spaltung D(+)-0,0-Dibenzoylweinsäure, so erhält man analog das Dibenzoyltartrat der rechtsdrehenden Base mit dem spezifischen Drehwert von
$[\alpha]_D^{20} = +77°$
und die Base mit dem spezifischen Drehwert
$[\alpha]_D^{20} = +89°$.

c) 2-(Benzylamino)methyl-1-(3-methoxyphenyl)-cyclohexanol

43,0g (0,103 Mol) 2-Dibenzylaminomethyl-1-(3-methoxy-phenyl)-cyclohexanol (hergestellt gemäss b)) werden in 300 ml Methanol gelöst und mit 10g 5%igem Palladium-Kohle-Katalysator bei Normaldruck und Raumtemperatur hydriert. Nach Aufnahme von einem Äquivalent Wasserstoff kommt die Hydrierung zum Stillstand. Man saugt vom Katalysator ab, dampft die Lösung ein und destilliert das Produkt im Vakuum.
Ausbeute: 25g (73% der Theorie)
Kp 180–190°C/0,07 mbar.

In gleicher Weise wurden erhalten:

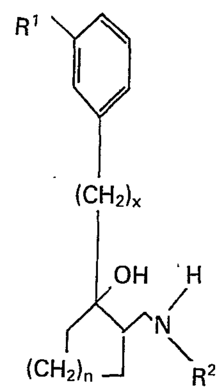

| $R^1$ | $R^2$ | x | n | Ausbeute % | Kp (°C)/mbar |
|---|---|---|---|---|---|
| H | $CH_3$ | 0 | 2 | 95 | 130–135/0,7 |
| $CH_3O$ | $CH_3$ | 0 | 2 | 91 | 160/0,07 |
| H | $CH_3$ | 1 | 2 | 87 | 150–/0,7 |
| $CH_3O$ | $CH_3$ | 1 | 2 | 94 | 170–180/0,07 |
| $CH_3O$ | $CH_3$ | 0 | 1 | 57 | 130–140/0,013 |
| $CH_3O$ | $CH_3$ | 0 | 3 | 61 | 150–160/0,013 |

d) 2-(N-Chloracetyl)methylaminomethyl-1-(3-methoxyphenyl)-cyclohexanol

Zu einer Lösung von 30g (0,12 Mol) 2-Methylaminomethyl-1-(3-methoxyphenyl)-cyclohexanol (vgl. c)) in 300 ml Ether gibt man 100 ml 2-N-Natronlauge und tropft dann innerhalb von 15 min unter Rühren 17g (0,15 Mol) Chloracetylchlorid zu. Man erwärmt anschliessend 30 min, kühlt, trennt die Etherschicht ab, trocknet mit Natriumsulfat und destilliert das Lösungsmittel ab. Der Rückstand wird roh weiterverarbeitet.
Ausbeute: ca. 100% der Theorie.

Auf die gleiche Weise wurden erhalten:

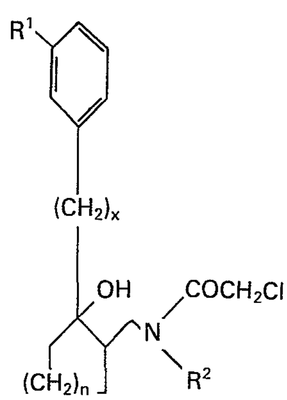

| | $R^1$ | $R^2$ | x | n |
|---|---|---|---|---|
| 1 | H | $CH_3$ | 0 | 2 |
| 2 | H | $CH_3$ | 1 | 2 |
| 3 | $CH_3O$ | $CH_3$ | 1 | 2 |
| 4 | $CH_3O$ | $CH_3$ | 0 | 1 |
| 5 | $CH_3O$ | $CH_3$ | 0 | 3 |
| 6 | $CH_3O$ | $CH_2$- 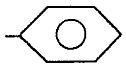 | 0 | 2 |

e) 4-Methyl-9a-(3-methoxyphenyl)-perhydro-1,4-benzoxazepin-3-on

16g (0,05 Mol) des in c) beschriebenen 2-(N-Chloracetyl)methylaminomethyl-1-(3-methoxyphenyl)-cyclohexanols werden in 200ml Dimethylsulfoxid unter Rühren gelöst und im Verlauf von 30min unter leichter Kühlung bei 20°C 10g Kalium-tert.-butanolat portionsweise zugegeben. Man erwärmt anschliessend 30min auf 50°C und lässt über Nacht bei Raumtemperatur weiterrühren. Zur Aufarbeitung wird das Dimethylsulfoxid im Vakuum abdestilliert, der Rückstand in Wasser aufgenommen, mit Methylenchlorid extrahiert, die organische Phase abgetrennt, getrocknet und die Lösung eingedampft.

Der Rückstand wird aus 2 Teilen Isopropanol umkristallisiert.
Ausbeute: 8,5g (65% der Theorie)
Fp. 120–121°C
Kp. 195–200/0,07 mbar.

Auf die gleiche Weise wurden die nachstehenden Substanzen erhalten, die jedoch alle nicht kristallin erhalten werden konnten.

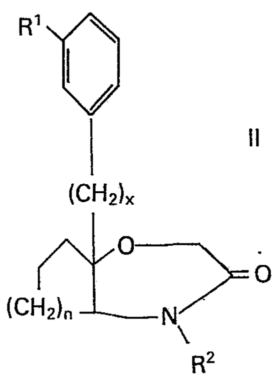

II

| | $R^1$ | $R^2$ | x | n | Ausbeute |
|---|---|---|---|---|---|
| 1 | H | $CH_3$ | 0 | 2 | 100 (roh) |
| 2 | H | $CH_3$ | 1 | 2 | 81 (roh) |
| 3 | $CH_3O$ | $CH_3$ | 1 | 2 | 100 (roh) |
| 4 | $CH_3O$ | $CH_3$ | 0 | 1 | 65 (roh) |
| 5 | $CH_3O$ | $CH_3$ | 0 | 3 | 100 (roh) |
| 6 | $CH_3O$ | $CH_2$- 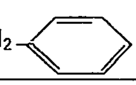 | 0 | 2 | 100 (roh) |

Herstellung der Endprodukte

Beispiel 1

4-Methyl-9a-(3-methoxybenzyl)-perhydro-1,4-benzoxazepin

40,8g (0,14 Mol) rohes 4-Methyl-9a-(3-methoxybenzyl-perhydro-1,4-benzoxazepin-3-on (vgl. e)) werden in 100ml absolutem Tetrahydrofuran gelöst und bei Rückflusstemperatur langsam zu einer siedenden Suspension von 9,5g (0,25 Mol) Lithiumaluminiumhydrid in 250ml Tetrahydrofuran zugetropft. Man hält die Lösung anschliessend unter Stickstoff 6 Stunden am Sieden, kühlt ab, versetzt mit wenig Wasser und erhält nach Abdestillieren des Lösungsmittels die rohe Base, die destilliert wird.

Die reine Base wird mit isopropanolischer Salzsäure in ihr Hydrochlorid überführt.
Ausbeute (Base) 24,5g (60% der Theorie)
Kp. 190–210°C/0,27mbar
Hydrochlorid: 220–221°C.

In ähnlicher Weise wurden die tabellarisch aufgeführten Substanzen erhalten:

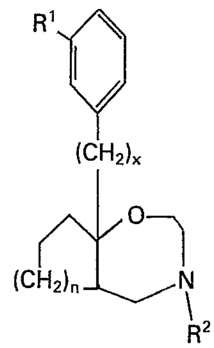

| Beispiel | $R^1$ | $R^2$ | x | n | Ausbeute % | Hydrochlorid Fp. °C |
|---|---|---|---|---|---|---|
| 2 | H | $CH_3$ | 0 | 2 | 67 | 250 |
| 3 | $CH_3O$ | $CH_3$ | 0 | 2 | 74 | 214 |
| 4 | H | $CH_3$ | 1 | 2 | 58 | 251 |
| 5 | $CH_3O$ | $CH_3$ | 0 | 3 | 55 | 208–10 |
| 6 | $CH_3O$ | $CH_3$ | 0 | 1 | 46 | 218–20 |

Beispiel 7

α) 4-Methyl-9a-(3-hydroxyphenyl)-perhydro-1,4-benzoxazepin

Man bereitet aus 2,3g (0,1 Mol) Natrium, 100ml absolutem Ethanol und 6,2g (0,1 Mol) Ethylmercaptan eine ethanolische Lösung von Natriumethylmercaptid, destilliert dann im Vakuum den Alkohol ab, fügt 50ml trockenes Dimethylformamid und 5,5g (0,02 Mol) 4-Methyl-9a-(3-methoxyphenyl)-perhydro-1,4-benzoxazepin (erhalten nach Beispiel 1) hinzu und erhitzt drei Stunden auf 140°C. Anschliessend verdünnt man mit 500ml Wasser, neutralisiert mit Essigsäure und extrahiert die Lösung mehrmals mit Methylenchlorid. Nach Entfernen des Lösungsmittels nimmt man in 100ml Ether auf und fällt die Substanz als Hydrochlorid durch Einleiten von Chlorwasserstoffgas. Es wird aus Isopropanol umkristallisiert.
Ausbeute: 4,6g (77% der Theorie)
Fp. 230°C.

β) 4-Methyl-9a-(3-acetoxyphenyl)-perhydro-1,4-benzoxazepin

3,0g (0,1 Mol) der so erhaltenen Substanz werden mit 50ml Essigsäureanhydrid 3 Stunden zum Sieden unter Rückfluss erhitzt. Anschliessend destilliert man das überschüssige Essigsäureanhydrid unter vermindertem Druck ab und kristallisiert den Rückstand aus wenig Isopropanol um. Man erhält die Substanz als Hydrochlorid.
Ausbeute: 2,8g (82% der Theorie)
Fp. 210°C.

Beispiel 8

α) 9a-(3-Methoxyphenyl)-perhydro-1,4-benzoxazepin

16g (0,045 Mol) analog Beispiel 1 erhaltenes 4-Benzyl-9a-(3-methoxyphenyl)perhydro-1,4-benzoxazepin (Kp. 240–260°C/0,07mbar) werden in üblicher Weise mit Palladium-Kohle-Katalysator (5g 5% Pd auf Kohle) in Eisessig hydriert, wobei man nach Filtration und Destillation 8,8g (75% der Theorie) 9a-(3-Methoxyphenyl)-perhydro-1,4-benzoxazepin mit einem Siedepunkt von 180–185°C/0,07mbar erhält.

β) 4-Allyl-9a-(3-methoxyphenyl)-perhydro-1,4-benzoxazepin

Die so erhaltene Verbindung wird mit 80ml Methylisobutylketon, 3,2g Allylbromid und 3,6g pulverisiertem Kaliumcarbonat 6 Stunden unter Rühren zum Sieden unter Rückfluss erhitzt. Man versetzt mit 100ml Wasser, trennt die organische Schicht ab, entfernt das Lösungsmittel, nimmt in Ether auf und fällt die Substanz als Hydrochlorid durch Einleiten von gasförmigem Chlorwasserstoff.

Nach Umkristallisieren aus Isopropanol erhält man 6,4g (57% der Theorie) des Hydrochlorids vom Schmelzpunkt 225°C.

Auf ähnliche Weise wird unter Verwendung von Ethylbromid 4-Ethyl-9a-(3-methoxyphenyl)-perhydro-1,4-benzoxazepin erhalten.
Ausbeute: 52% der Theorie
Fp. 195–196°C.

Beispiel 9

(+)- und (−)-4-Methyl-9a-(3-methoxyphenyl)-perhydro-1,4-benzoxazepin

Mit dem nach b) hergestellten (+)- und (−)-2-(N-Benzyl-methylamino)methyl-1-(3-methoxyphenyl)-cyclohexanol werden unter Anwendung der Verfahren c, d, e und nach Beispiel 1 die beiden optischen Antipoden von 4-Methyl-9a-(3-methoxy-phenyl)-perhydro-1,4-benzoxazepin hergestellt.

Spezifische Drehwerte (gemessen in Methanol C = 20mg/ml):
Basen: $[\alpha]^{20}_{D} = \pm 15°$
Hydrochloride: $[\alpha]^{20}_{D} = \pm 35°$
Schmelzpunkte: 190–191°C.

Beispiel 10

Auf einer Tablettenpresse werden in üblicher Weise Tabletten folgender Zusammensetzung gepresst:

20,00mg 4-Methyl-9a-(3-methoxyphenyl)-perhydro-1,4-benzoxazepin
50,00mg Maisstärke
4,50mg Gelatine
15,00mg Milchzucker
7,50mg Talk
0,75mg Aerosil® (chemisch reine Kieselsäure in submikroskopisch feiner Verteilung)
2,25mg Kartoffelstärke (als 6%iger Kleister)

Beispiel 11

In üblicher Weise werden Dragees folgender Zusammensetzung hergestellt:
20,00mg 4-Methyl-9a-(3-acetoxyphenyl)-perhydro-1,4-benzoxazepin
50,00mg Kernmasse
40,00mg Verzuckerungsmasse

Die Kernmasse besteht aus 9 Teilen Maisstärke, 3 Teilen Milchzucker und 1 Teil Luviskol® VA 64 (Vinylpyrrolidon-Vinylacetat-Mischpolymerisat 60:40, vgl. Pharm. Ind. 1962, 586). Die Verzuckerungsmasse besteht aus 5 Teilen Rohrzucker, 2 Teilen Maisstärke, 2 Teilen Calciumcarbonat und 1 Teil Talk. Die so hergestellten Dragees werden anschliessend mit einem magensaftresistenten Überzug versehen.

Beispiel 12

Es werden 10g 4-Methyl-9a-(3-methoxybenzyl)-perhydro-1,4-benzoxazepin-Hydrochlorid in 2,0l Wasser gelöst, mit Kochsalz isotonisch eingestellt und in 2ml fassende Ampullen steril abgefüllt.

**Patentansprüche für die Vertragsstaaten: BE, CH, DE, FR, GB, IT, LU, NL, SE**

1. 6,7-Cycloalkano-1,4-oxazepine der allgemeinen Formel I

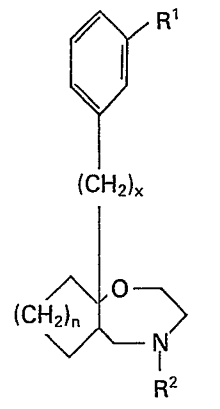

I,

worin $R^1$ ein Wasserstoffatom, eine Hydroxygruppe oder eine Alkoxy- oder Acyloxygruppe mit 1–4 Kohlenstoffatomen, $R^2$ einen Methyl-, Ethyl- oder Allylrest, n die Zahl 1, 2 oder 3 und x die Zahl 0 oder 1 bedeuten, sowie deren Salze mit physiologisch verträglichen Säuren.

2. 4-Methyl-9a-(3-methoxyphenyl)-perhydro-1,4-benzoxazepin und seine Salze mit physiologisch verträglichen Säuren.

3. 4-Methyl-8a-(3-methoxyphenyl)-perhydro-1,4-cyclopentoxazepin und seine Salze mit physiologisch verträglichen Säuren.

4. 4-Methyl-9a-(3-hydroxyphenyl)-perhydro-1,4-benzoxazepin und seine Salze mit physiologisch verträglichen Säuren.

5. 4-Methyl-9a-(3-acetoxyphenyl)-perhydro-1,4-benzoxazepin und seine Salze mit physiologisch verträglichen Salzen.

6. Verbindungen nach Anspruch 1 zur Verwendung bei der Bekämpfung von Schmerzzuständen.

7. Verfahren zur Herstellung von 6,7-Cycloalkano-1,4-oxazepinen der allgemeinen Formel I nach Anspruch 1, dadurch gekennzeichnet, dass man eine Verbindung der allgemeinen Formel II

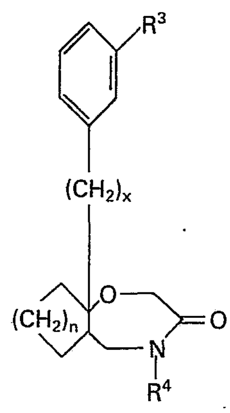

II,

worin $R^3$ ein Wasserstoffatom oder eine Alkoxygruppe mit 1–4 Kohlenstoffatomen, $R^4$ einen Methyl-, Ethyl- oder Allylrest oder eine Benzylgruppe darstellen, und x und n die in Anspruch 1 genannte Bedeutung haben reduziert und anschliessend gegebenenfalls eine Alkoxygruppe gegen eine Hydroxy- oder Acyloxygruppe und/oder eine Benzylgruppe gegen einen Kohlenwasserstoffrest $R^2$, der wie in Anspruch 1 definiert ist, austauscht und die so erhaltenen Verbindungen, falls gewünscht, in ihre Salze mit physiologisch verträglichen Säuren überführt.

8. Arzneimittel, enthaltend eine Verbindung gemäss Anspruch 1.

**Patentanspruch für den Vertragsstaat: AT**

Verfahren zur Herstellung von 6,7-Cycloalkano-1,4-oxazepinen der allgemeinen Formel I

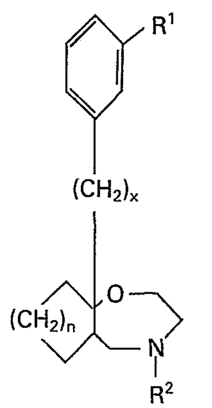

I,

worin $R^1$ ein Wasserstoffatom, eine Hydroxygruppe oder eine Alkoxy- oder Acyloxygruppe mit 1–4 Kohlenstoffatomen, $R^2$ einen Methyl-, Ethyl- oder Allylrest, n die Zahl 1, 2 oder 3 und x die Zahl 0 oder 1 bedeuten, sowie deren Salzen mit physiologisch verträglichen Säuren, dadurch gekennzeichnet, dass man eine Verbindung der allgemeinen Formel II

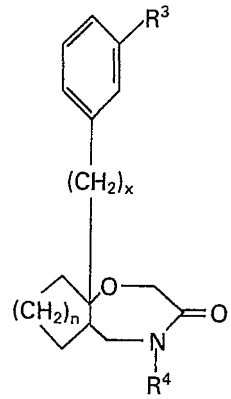

II,

worin $R^3$ ein Wasserstoffatom oder eine Alkoxygruppe mit 1–4 Kohlenstoffatomen, $R^4$ einen Methyl-, Ethyl- oder Allylrest oder eine Benzylgruppe darstellen und x und n wie in Formel I definiert sind, reduziert und anschliessend gegebenenfalls eine Alkoxygruppe gegen eine Hydroxy- oder Acyloxygruppe $R^3$ und/oder eine Benzylgruppe gegen einen Kohlenwasserstoffrest $R^2$ austauscht und die so erhaltenen Verbindungen, falls gewünscht, in ihre Salze mit physiologisch verträglichen Säuren überführt.

**Claims for the Contracting States: BE, CH, DE, FR, GB, IT, LU, NL, SE**

1. A 6,7-cycloalkano-1,4-oxazepine of the general formula I

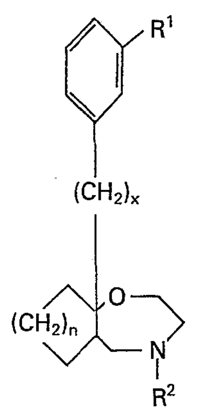

I,

where $R^1$ is hydrogen, hydroxyl or alkoxy or acyloxy of 1 to 4 carbon atoms, $R^2$ is methyl, ethyl or allyl, n is 1, 2 or 3, and x is 0 or 1, and its salts with physiologically acceptable acids.

2. 4-Methyl-9a-(3-methoxyphenyl)-perhydro-1,4-benzoxazepine and its salts with physiologically acceptable acids.

3. 4-Methyl-8a-(3-methoxyphenyl)-perhydro-1,4-cyclopentoxazepine and its salts with physiologically acceptable acids.

4. 4-Methyl-9a-(3-hydroxyphenyl)-perhydro-1,4-benzoxazepine and its salts with physiologically acceptable acids.

5. 4-Methyl-9a-(3-acetoxyphenyl)-perhydro-1,4-benzoxazepine and its salts with physiologically acceptable acids.

6. A compound as claimed in claim 1 for use in the treatment of algias.

7. A process for the preparation of a 6,7-cycloalkano-1,4-oxazepine of the general formula I as claimed in claim 1, characterized in that a compound of the general formula II

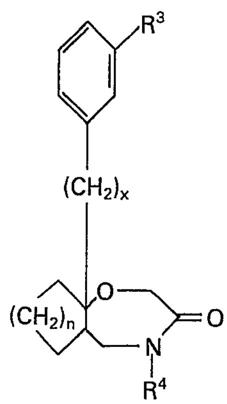

II,

where $R^3$ is hydrogen or alkoxy of 1 to 4 carbon atoms and $R^4$ is methyl, ethyl or allyl, or benzyl, and x and n have the meanings given in claim 1, is reduced, after which, where appropriate, alkoxy is replaced by hydroxyl or acyloxy and/or benzyl is replaced by a hydrocarbon radical $R^2$ as defined in claim 1, and, if desired, the resulting compound is converted into a salt with a physiologically acceptable acid.

8. A drug containing a compound as claimed in claim 1.

**Claim for the contracting State: AT**

A process for the preparation of a 1,4-cycloalkano-oxazepine of the general formula I

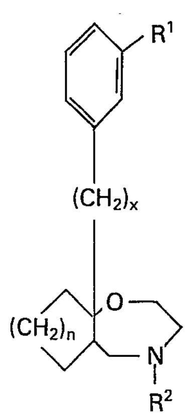

I,

where $R^1$ is hydrogen, hydroxyl or alkoxy or acyloxy of 1 to 4 carbon atoms, $R^2$ is methyl, ethyl or allyl, n is 1, 2 or 3, and x is 0 or 1, and its salts with physiologically acceptable acids, characterized in that a compound of the general formula II

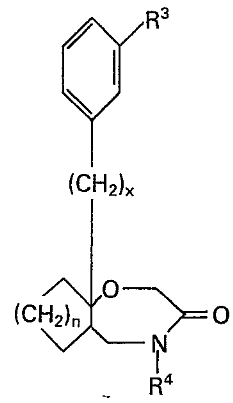

II,

where $R^3$ is hydrogen or alkoxy of 1 to 4 carbon atoms and $R^4$ is methyl, ethyl or allyl, or benzyl, is reduced, after which, where appropriate, alkoxy is replaced by hydroxyl or acyloxy and/or benzyl is replaced by a hydrocarbon radical and, if desired, the resulting compound is converted into a salt with a physiologically acceptable acid.

**Revendications pour les Etats contractants: BE, CH, DE, FR, GB, IT, LU, NL, SE**

1. 6,7-cycloalcano-1,4-oxazépines de formule générale I

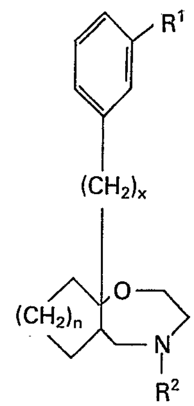

I,

dans laquelle $R^1$ représente un atome d'hydrogène, un groupe hydroxy ou un groupe alcoxy ou acyloxy à 1–4 atomes de carbone, $R^2$ un reste méthyle, éthyle ou allyle, n le nombre 1, 2 ou 3 et x le nombre 0 ou 1, ainsi que leurs sels avec des acides physiologiquement compatibles.

2. 4-méthyl-9a-(3-méthoxyphényl)-perhydro-1,4-benzoxazépine et ses sels avec des acides physiologiquement compatibles.

3. 4-méthyl-8a-(3-méthoxyphényl)-perhydro-1,4-cyclopentoxazépine et ses sels avec des acides physiologiquement compatibles.

4. 4-méthyl-9a-(3-hydroxyphényl)-perhydro-1,4-benzoxazépine et ses sels avec des acides physiologiquement compatibles.

5. 4-méthyl-9a-(3-acétoxyphényl)-perhydro-1,4-benzoxazépine et ses sels avec des acides physiologiquement compatibles.

6. Composés selon la revendications 1 pour utilisation lors de la lutte contre des états douloureux.

7. Procédé de préparation de 6,7-cycloalcano-1,4-oxazépines de formule générale I selon la revendication 1, caractérisé par le fait que l'on réduit un composé de formule générale II

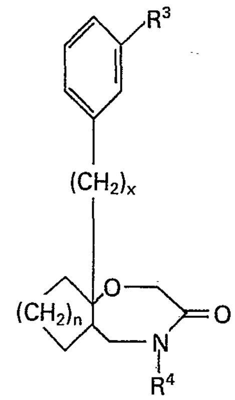

II,

dans laquelle $R^3$ représente un atome d'hydrogène ou un groupe alcoxy à 1–4 atomes de carbone, $R^4$ un reste méthyle, éthyle, ou allyle ou un groupe benzyle, et x et n ont la signification indiquée à la revendication 1, puis, éventuellement, on échange un groupe alcoxy contre un groupe hydroxy ou acyloxy et/ou un groupe benzyle contre un reste d'hydrocarbure $R^2$ qui est défini comme à la revendication 1 et l'on transforme les composés ainsi obtenus, si on le désire, en leurs sels avec les acides physiologiquement compatibles.

8. Médicament contenant un composé selon la revendication 1.

**Revendication pour l'Etat contractant: AT**

Procédé de préparation de 1,4-cycloalcanooxazépines de formule générale I

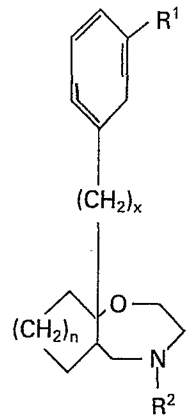

I,

dans laquelle $R^1$ représente un atome d'hydrogène, un groupe hydroxy ou un groupe alcoxy ou acyloxy à 1–4 atomes de carbone, $R^2$ un reste méthyle, éthyle ou allyle, n le nombre 1, 2 ou 3 et x le nombre 0 ou 1, ainsi que leurs sels avec des acides physiologiquement compatibles, caractérisé par le fait que l'on réduit un composé de formule II

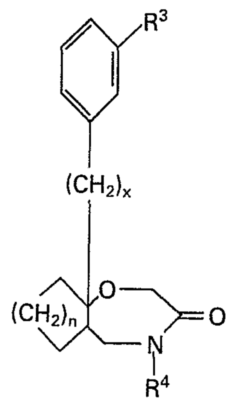

II,

dans laquelle $R^3$ représente un atome d'hydrogène ou un groupe alcoxy à 1–4 atomes de carbone, $R^4$ un reste méthyle, éthyle, ou allyle ou un groupe benzyle, puis, éventuellement, on échange un groupe alcoxy contre un groupe hydroxy ou acyloxy et/ou un groupe benzyle contre un reste d'hydrocarbure et l'on transforme les composés ainsi obtenus, si on le désire, en leurs sels avec des acides physiologiquement compatibles.